(19) Europäisches Patentamt / European Patent Office / Office européen des brevets

(11) **EP 3 842 416 B1**

(12) **EUROPEAN PATENT SPECIFICATION**

(45) Date of publication and mention
of the grant of the patent:
**01.03.2023 Bulletin 2023/09**

(21) Application number: **19852381.3**

(22) Date of filing: **16.08.2019**

(51) International Patent Classification (IPC):
*C07C 265/04* (2006.01)   *C07C 231/10* (2006.01)
*C07C 233/18* (2006.01)   *C07C 263/10* (2006.01)
*C07C 263/20* (2006.01)   *C07C 269/02* (2006.01)
*C07C 271/16* (2006.01)   *C07C 271/60* (2006.01)
*C07C 333/04* (2006.01)   *C07D 231/12* (2006.01)
*C08G 18/81* (2006.01)   *C07B 61/00* (2006.01)

(52) Cooperative Patent Classification (CPC):
(C-Sets available)
**C08G 18/4833; C07C 231/10; C07C 263/10;
C07C 263/20; C07C 269/02; C08G 18/3876;
C08G 18/672; C08G 18/8116**   (Cont.)

(86) International application number:
**PCT/JP2019/032136**

(87) International publication number:
**WO 2020/040050 (27.02.2020 Gazette 2020/09)**

(54) **COMPOSITION, PRODUCTION METHOD FOR COMPOSITION, AND PRODUCTION METHOD FOR UNSATURATED COMPOUND**

ZUSAMMENSETZUNG, HERSTELLUNGSVERFAHREN FÜR DIE ZUSAMMENSETZUNG UND HERSTELLUNGSVERFAHREN FÜR UNGESÄTTIGTE VERBINDUNGEN

COMPOSITION, PROCÉDÉ DE PRODUCTION CORRESPONDANT, ET PROCÉDÉ DE PRODUCTION DE COMPOSÉ INSATURÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2018 JP 2018154148**

(43) Date of publication of application:
**30.06.2021 Bulletin 2021/26**

(73) Proprietor: **Showa Denko K.K.**
**Tokyo 105-8518 (JP)**

(72) Inventors:
• **NISHIMURA, Norihito**
  **Tokyo 105-8518 (JP)**
• **OHNO, Katsutoshi**
  **Tokyo 105-8518 (JP)**

(74) Representative: **Strehl Schübel-Hopf & Partner**
**Maximilianstrasse 54**
**80538 München (DE)**

(56) References cited:
**WO-A1-2010/016540**   **JP-A- 2003 231 663**
**JP-A- 2016 150 922**   **KR-B1- 101 805 292**

(52) Cooperative Patent Classification (CPC): (Cont.)

C-Sets
**C07C 231/10, C07C 233/18;**
**C07C 263/10, C07C 265/04;**
**C07C 263/20, C07C 265/04;**
**C07C 269/02, C07C 271/16;**
**C07C 269/02, C07C 271/60**

**Description**

TECHNICAL FIELD

The invention is related to a composition containing an unsaturated isocyanate compound, a method of producing the composition, and a method of producing an unsaturated compound.

BACKGROUND TECHNOLOGY

Conventionally, a unsaturated compound such as an unsaturated urethane compound, an unsaturated thiourethane compound, an unsaturated urea compound, and an unsaturated amide compound, is produced by reacting an unsaturated isocyanate compound with a compound having active hydrogen (compound having functional group having active hydrogen). The unsaturated compound thus produced is used in various applications.

For example, there is an unsaturated urethane compound produced by reacting 2-methacryloyloxyethyl isocyanate (Hereinafter, this may be referred to as "MOI". An example of a specific product is "KarenzMOI (registered trademark)".) which is an unsaturated isocyanate compound with a polyalkylene glycol which is a compound having a hydroxyl group. There are known methods for producing the unsaturated isocyanate compound, such as a method for reacting an amine having an ethylenic double bond with phosgene to decompose the amine by heating (for example, see Patent Document 1). The unsaturated urethane compound is proposed to be used as a material for contact lenses (for example, see Patent Document 2), a material for solid solvents of polymer solid electrolytes (for example, see Patent Document 3.), and a material for immobilizing biological materials (for example, refer to Patent Documents 4 and 5).

Further, Patent Document 6 discloses an unsaturated urea compound obtained by reacting MOI with an organopolysiloxane having amino groups at both ends of the molecule. Patent Document 6 discloses using the unsaturated urea compound as a material for a radiation-curable adhesive organopolysiloxane composition.

Patent Document 7 discloses a urethane acrylate synthesized by reacting an unsaturated isocyanate compound such as MOI with a product obtained by reacting a dimer diol with a polyisocyanate. Further, Patent Document 7 discloses a curable composition containing the urethane acrylate.

Examples of the unsaturated isocyanate compound used as a material of the unsaturated compound include acryloyloxyethyl isocyanate (Hereinafter, this may be referred to as "AOI". An example of a specific product is "KarenzAOI (registered trademark)".) and methacryloyl isocyanate (Hereinafter referred to as "MAI".) in addition to MOI. MOI, AOI, and MAI are industrially manufactured, commercially available, and readily available.

MOI is synthesized by the reaction of isopropenyloxazoline or 2-aminoethyl methacrylate hydrochloride with phosgene. AOI is synthesized by the reaction of 2-vinyloxazoline or 2-aminoethylacrylate hydrochloride with phosgene. MAI is synthesized by the reaction of methacrylic amide with oxalyl chloride.

The unsaturated isocyanate compound synthesized as described above includes impurities such as by-products and catalyst residues. For this reason, after the unsaturated isocyanate compound is synthesized, an operation for removing impurities to increase the purity is generally performed (for example, see Patent Documents 8 and 9.).

The quality of the synthesized unsaturated isocyanate compound is conventionally determined by various methods. Specifically, there are a method for confirming the appearance of the unsaturated isocyanate compound such as the presence or absence of turbidity and hue, a method for confirming the purity of the unsaturated isocyanate compound by using gas chromatography, a method for confirming the hydrolyzable chlorine content in the unsaturated isocyanate compound by potentiometric titration, and a method for confirming a soluble impurity in the unsaturated isocyanate compound by using gel permeation chromatography (GPC) (see, for example, Patent Document 10.).

Generally, in order to stably transport and store the unsaturated isocyanate compound, a polymerization inhibitor is added to the unsaturated isocyanate compound. As the polymerization inhibitor, hydroquinone or the like is used and is added at a concentration of several tens to several hundred ppm. For example, Patent Document 7 discloses that, when an unsaturated urethane compound is synthesized using an unsaturated isocyanate compound, a polymerization inhibitor is added in an amount of 0.01 to 10 parts by mass based on 100 parts by mass of the total weight components.

[Patent Documents]

[Patent Document 1] U.S. Patent No. 2821544
[Patent Document 2] Japanese Patent Laid-Open No. H06-322051
[Patent Document 3] Japanese Patent Laid-Open No. H06-187822
[Patent Document 4] Japanese Patent Laid-Open No. S60-234582
[Patent Document 5] Japanese Patent Laid-Open No. S60-234583

[Patent Document 6] Japanese Patent Laid-Open No. H06-184256
[Patent Document 7] WO 2011/074503
[Patent Document 8] Japanese Patent No. 4273531
[Patent Document 9] Japanese Patent No. 4823546
[Patent Document 10] Japanese Patent Laid-Open No. 2007 -8828

[Non-Patent Documents]

[Non-Patent Document 1] CMC Technical Library "Polymer degradation mechanism and stabilization technology" CMC Publishing Co., Ltd. (Issued on 2005/04) Page 168, Figure 5.

SUMMARY OF THE INVENTION

Conventional unsaturated isocyanate compounds may cause unexpected viscosity increases or gelation during storage and/or transport, even though there is no significant difference in quality by using conventional determination methods. Therefore, it has been required to improve the stability during storage.
In addition, the conventional unsaturated isocyanate compound does not show a large difference in quality by using the conventional determination method, but when the unsaturated compound is produced by using the same, the viscosity of the reaction product is sometimes rapidly increased or gelatinized during production. Therefore, it has been required to improve the stability in use.
In order to improve the storage stability and the utilization stability of the unsaturated isocyanate compound, a sufficient amount of a polymerization inhibitor may be added to the unsaturated isocyanate compound.
However, when a large number of polymerization inhibitors are added to an unsaturated isocyanate compound, a colored component caused by the polymerization inhibitor is easily generated together with the unsaturated compound when the unsaturated compound is produced using the unsaturated isocyanate compound as a raw material (for example, see Non-patent Document 1.). Therefore, the produced unsaturated compound may be colored.
An object of the present invention is to provide a composition excellent in stability during storage and stability during use, and a method of producing the composition.
Another object of the present invention is to provide a method of producing an unsaturated compound unlikely gelatinized during production by using the composition as a raw material.

[Means to Solve the Problem]

In order to solve the above problems, the present inventors have diligently investigated. As a result, it was found that a compound having a specific structure (hereinafter referred to as "specific compound") contained as an impurity in the unsaturated isocyanate compound after purification is one of the causes of deterioration of the storage stability and the stability during use of the unsaturated isocyanate compound. Further, the inventors of the present invention have repeatedly studied and found that the concentration of the specific compound in the unsaturated isocyanate compound has a correlation with the viscosity increase and the occurrence of gelation of the unsaturated isocyanate compound during storage.
However, a purification process for removing the specific compound from the unsaturated isocyanate compound has not been known. Therefore, the present inventors have studied a purification process for removing the specific compound from an unsaturated isocyanate compound. As a result, it was found that the specific compound can be removed from the unsaturated isocyanate compound by purification by using a distillation process at a reflux ratio of 2.0 to 4.0, a pressure of 1.0 to 10.0 kPa, and a distillation temperature of 90 to 140°C.
Further, the inventors examined the viscosity increase and gelation during storage of the unsaturated isocyanate compound purified by the distillation process at the reflux ratio, the pressure and the distillation temperature. As a result, it has been found that the viscosity increase and gelation can be suppressed by setting the concentration of the specific compound in the unsaturated isocyanate compound to 0.2 parts by mass of less with respect to 100 parts by mass or less of the unsaturated isocyanate compound.
The inventors have found that even when the specific compound in the unsaturated isocyanate compound is sufficiently removed by the distillation process, the unsaturated isocyanate compounds after the distillation process are bonded to each other to form the specific compound. Further, the inventors have found that the increase of the specified compound in the unsaturated isocyanate compound after the distillation process can be effectively suppressed by performing a vacuum breaking step in which a gas having a dew point of -30°C or lower and containing nitrogen gas and oxygen gas at an oxygen concentration of 1 vol% or more and less than the critical oxygen concentration of the unsaturated isocyanate compound is supplied into a distillation apparatus after the distillation process to return the pressure in the distillation apparatus to the atmospheric pressure; and performing a filling step in which the purified product in the distillation apparatus after the distillation process is stored in a container and air having a dew point of -30°C or lower is filled in the gas phase portion in the container.

[Effect of the Invention]

The composition comprises a compound (A) represented by general formula (1) and a compound (B). The compound (B) is an oligomer in which two or more molecules of the compound (A) are bonded to each other by ethylenically unsaturated groups of each compound (A). The composition contains 0.00002 to 0.2 parts by mass of the compound (B) with respect to 100 parts by mass of the compound (A). Therefore, unexpected viscosity increase and gelation of the composition unlikely occur during storage and transportation, and stability during storage is excellent. The composition of the present invention unlikely causes a rapid increase in viscosity or gelation of a reaction product generated during production when an unsaturated compound is produced by using the composition, and is excellent in stability in use.

Since the composition of the present invention is excellent in stability during storage and stability during use, it is not necessary to contain a large amount of a polymerization inhibitor which produces a colored component. Therefore, it is possible to prevent the unsaturated compound produced by using the composition of the present invention from being colored by the coloring component caused by the polymerization inhibitor.

Further, since the composition of the present invention contains 0.00002 parts by mass or more of the compound (B) with respect to 100 parts by mass of the compound (A), the unsaturated compound can be produced in a high yield.

The method of producing a composition according to the present invention comprises steps of: producing a mixture containing a compound (A) represented by general formula (1) and a compound (B); and purifying the mixture by a distillation process at a reflux ratio of 2.0 to 4.0, a pressure of 1.0 to 10.0 kPa, and a distillation temperature of 90 to 140°C to obtain a purified product. The compound (B) is an oligomer in which two or more molecules of the compound (A) are bonded to each other by ethylenically unsaturated groups of each compound (A). The mixture contains more than 0.2 parts by mass of the compound (B) with respect to 100 parts by mass of the compound (A). The purified product contains 0.00002 to 0.2 parts by mass of the compound (B) with respect to 100 parts by mass of the compound (A). Accordingly, the composition of the present invention, in which the compound (B) is sufficiently removed from the mixture, can be produced in high yield, wherein the compound (B) affects the stability during storage and the stability during use.

Further, in the method of producing the composition of the present invention, the purification step includes a vacuum breaking step of supplying a gas having a dew point of -30°C or lower containing nitrogen gas and oxygen gas at an oxygen concentration of 1 vol% or more and less than the critical oxygen concentration of the compound (A) into the distillation apparatus after distillation by the distillation process and returning the pressure in the distillation apparatus to atmospheric pressure, and a filling step of storing the purified product in the distillation apparatus after distillation process into a container and filling the gas phase portion in the container with air having a dew point of -30°C or lower. Therefore, the ethylenically unsaturated groups of the compound (A) unlikely bond to each other in the purified product after the distillation process, and the formation or increase of the compound (B) in the purified product can be prevented. Therefore, according to the method of producing the composition of the present invention, a composition having good stability during storage and stability during use can be obtained.

The method of producing the unsaturated compound includes steps of mixing the composition of the invention with a compound having active hydrogen and reacting the compound (A) contained in the composition with the compound having active hydrogen to obtain a reaction product. In the method of producing the unsaturated compound of the present invention, since the composition used as a material contains 0.00002 to 0.2 parts by mass of the compound (B) with respect to 100 parts by mass of the compound (A), rapid increase in viscosity or gelation of the reaction product is unlikely generated during production, and excellent productivity is obtained.

MODE FOR CARRYING OUT THE INVENTION

Preferred examples of the present invention will be described below.

The pressure described herein is an absolute pressure.

"Composition"

The composition of this embodiment contains an unsaturated isocyanate compound. The composition of this embodiment comprises a compound (A) represented by general formula (1) and a compound (B). The compound (B) is an oligomer in which two or more molecules of the compound (A) are bonded to each other by ethylenically unsaturated groups of each compound (A). The composition of the present embodiment contains 0.00002 to 0.2 parts by mass of the compound (B) with respect to 100 parts by mass of the compound (A). In the composition of the present embodiment, the content of the compound (A) in the composition is preferably 95.0% by mass or more.

$$(R_1\text{-COO})_n\text{-}R_2\text{-(NCO)}_m \qquad (1)$$

(In general formula (1), $R_1$ is an ethylenically unsaturated group having 2 to 7 carbon atoms. $R_2$ is a m + n valent hydrocarbon group having 1 to 7 carbon and optionally contain an ether group. And, n and m are integers of 1 of 2.)

In general formula (1), $R_1$ is an ethylenically unsaturated group having 2 to 7 carbon atoms. The ethylenically unsaturated bond of $R_1$ may be one of two or more. When the number of carbon atoms is 6 or more, the reactivity of the ethylenically unsaturated group is lowered, so that $R_1$ is preferably an ethylenically unsaturated group having 2 to 5 carbon atoms. The carbon number is preferably 2 to 3 or 4 to 5. Among the ethylenically unsaturated groups having 2 to 5 carbon atoms, particularly, $CH_2=C(CH_3)$- or a vinyl group is preferably used because the raw material is easily available.

In general formula (1), $R_2$ is an m + n valent hydrocarbon group having 1 to 7 carbon atoms and the hydrocarbon group may be a linear or branched chain. The carbon number of the m + n valent hydrocarbon group represented by $R_2$ is preferably 2 to 4, and more preferably 2. $R_2$ may contain an ether group. $R_2$ is preferably $-CH_2CH_2-$, $-CH_2-$, or $-CH_2CH_2OCH_2CH_2-$ in view of the availability of the raw materials.

In general formula (1), n and m are integers of 1 or 2, and both are preferably 1 in view of ease of synthesis.

As the compound (A) represented by general formula (1), for example, at least one compound selected from the group consisting of 2-methacryloyloxyethyl isocyanate, 3-methacryloyloxy-n-propyl isocyanate, 2-methacryloyloxyisopropyl isocyanate, 4-methacryloyloxy-n-butyl isocyanate, 2-methacryloyloxy-tert-butyl isocyanate, 2-methacryloyloxybutyl-4-isocyanate, 2-methacryloyloxybutyl-3-isocyanate, 2-methacryloyloxybutyl-2-isocyanate, 2-methacryloyloxybutyl-1-isocyanate, 5-methacryloyloxy-n-pentyl isocyanate, 6-methacryloyloxy- n-hexyl isocyanate, 7-methacryloyloxyn-heptylisocyanate, 2-(isocyanatoethyloxy) ethyl methacrylate, 3-methacryloyloxyphenyl isocyanate, 2-acryloyloxyethyl isocyanate, and 2-acryloyloxyisocyanate, 3-acryloyloxy-n-propyl isocyanate, 2-acryloyloxyisopropyl isocyanate, 4-acryloyloxy-n-butyl isocyanate, 2-acryloyloxy-tert-butyl isocyanate, 2-acryloyloxybutyl -4 isocyanate, 2-acryloyloxybutyl-3-isocyanate, 2-acryloyloxybutyl-2-isocyanate, 2-acryloyloxybutyl-1-isocyanate, 5-acryloyloxy-n-pentyl isocyanate, 6-acryloyloxy-n-hexyl isocyanate, 7-acryloyloxy-n-heptyl isocyanate, 2-(isocyanatoethyloxy) ethyl acrylate, 3-acryloyloxyphenyl isocyanate, 4-acryloyloxyphenyl isocyanate, 1,1-bis (methacryloyloxymethyl) methyl isocyanate, 1,1-bis (methacryloyloxymethyl) ethyl isocyanate, 1,1-bis (acryloyloxymethyl) methyl isocyanate, 1,1-bis (acryloyloxymethyl) ethyl isocyanate, and 2'-pentenoyl-4-oxyphenyl isocyanate is used. Among these compounds, especially in view of ease of synthesis and availability of raw materials, the compound (A) is preferably 2-methacryloyloxyethyl isocyanate (examples of specific products: KarenzMOI (registered trademark)), 2-acryloyloxyethyl isocyanate (example of specific product: KarenzAOI (registered trademark)), 2-(isocyanatoethyloxy)ethyl methacrylate (example of specific product: KarenzMOI-EG (registered trademark)), 2-(isocyanatoethyloxy)ethyl acrylate (example of specific product: KarenzAOI-EG), of 1,1-bis(methacryloyloxymethyl)ethyl isocyanate (example of specific product: KarenzBEI (registered trademark)). It should be noted that the products including Karenz in the registered trademark described herein are available from Showa Denko K.K.

The content of the compound (A) in the composition of the present embodiment is not particularly limited, and may be, for example, from 90.0% by mass to less than 95.0% by mass, and may be 95.0% by mass or more. The content of the compound (A) in the composition of this embodiment is preferably 95.0% by mass or more, more preferably 97.0% by mass or more, and still more preferably 98.0% by mass to 99.9% by mass. When the content of the compound (A) in the composition is 95.0% by mass or more, it can be suitably used as a raw material for producing an unsaturated compound. When the content of the compound (A) is 99.9% by mass or less, the composition can be efficiently produced by a method of purification by a distillation process, which is preferable. The content of the compound (A) in the composition of the present embodiment is not limited to the above, and can be optionally selected as necessary. For example, the amount of the lower limit of the content of the compound (A) may be 50% by mass or more, 70% by mass or more, or 80% by mass or more.

The compound (B) is an oligomer in which two or more molecules of the compound (A) are bonded with ethylenically unsaturated groups of each compound (A). The compound (B) is presumed to be an impurity which is by-produced when the compound (A) represented by the general formula (1) is produced by a production method described later. The compound (B) degrades the stability of the composition during storage and utilization. The oligomer is a dimer to 100 mer of the compound (A).

In the present embodiment, with respect to 100 parts by mass of the compound (A), 0.00002 to 0.2 parts by mass of the compound (B) are contained in the composition. Since the content of the compound (B) in the composition is 0.2 parts by mass or less with respect to 100 parts by mass of the compound (A), excellent stability in storage and stability in use can be obtained. With respect to 100 parts by mass of the compound (A), the content of the compound (B) in the composition is preferably 0.1 parts by mass or less, more preferably 0.05 parts by mass or less in order to further improve the stability during storage and the stability during use. Further, since the content of the compound (B) in the composition is 0.00002 parts by mass or more with respect to 100 parts by mass of the compound (A), a yield in producing the compound (A) can be ensured, and the composition can be produced in a high yield. The content of the compound (B) in the composition is preferably 0.0002 parts by mass or more with respect to 100 parts by mass of the compound (A) in order to further improve the yield of the compound (A).

The composition of the present embodiment may contain an additive in addition to the compound (A) and the compound (B) to the extent that the effect of the present invention is not impaired. Examples of the additive include a polymerization inhibitor such as hydroquinone.

The composition of the present embodiment is preferably contained in a container filled with air having a dew point of -30°C or lower in a gas phase portion. This makes it difficult to bond the ethylenically unsaturated groups of the compound (A) in the composition contained in the container. As a result, the formation or increase of the compound (B) in the composition can be prevented, and the stability of the composition during storage and during use can be improved.

The composition of the present embodiment is preferably contained in an air-permeable container. Examples of the air-permeable container include a container having an air-permeable inner bag formed of polyethylene (PE). When the composition is contained in a container having air permeability, bonding between ethylenically unsaturated groups of the compound (A) is less likely to occur in the composition, and formation or increase of the compound (B) in the composition can be prevented.

"Method for Producing Composition"

The method of producing the composition of the present embodiment is a method of producing the composition contained in a container.

The method of producing a composition of the present embodiment includes a step of producing a mixture containing a compound (A) represented by the general formula (1) and a compound (B), and a step of purifying the mixture by a distillation process at a reflux ratio of 2.0 to 4.0, a pressure of 1.0 to 10.0 kPa, and a distillation temperature of 90 to 140°C to obtain a purified product. The compound (B) is an oligomer in which two or more molecules of the compound (A) are bonded to each other by ethylenically unsaturated groups of each compound (A). The mixture contains more than 0.2 parts by mass of the compound (B) with respect to 100 parts by mass of the compound (A). The purified product contains 0.00002 to 0.2 parts by mass of the compound (B) with respect to 100 parts by mass of the compound (A). The purified product preferably has a content of the compound (A) of 95.0% by mass or more.

(Step of Producing Mixture)

As a method of producing the mixture containing the compound (A) and the compound (B), for example, a method of producing the compound (A) simultaneously with producing the compound (B) by using a conventionally known method of producing the compound (A) may be used.

Specifically, for example, an unsaturated carboxylic acid aminoalkyl ester hydrochloride is synthesized by the reaction of an unsaturated carboxylic acid chloride with an amino alcohol hydrochloride. Next, an unsaturated carboxylic acid isocyanatoalkyl ester as a compound (A) is produced by reacting an unsaturated carboxylic acid aminoalkyl ester hydrochloride with carbonyl chloride. At the same time, a method for by-producing a compound (B) in which two or more molecules of the compound (A) are oligomers formed by bonding ethylenically unsaturated groups of each compound (A) with each other can be used.

The mixture containing the compound (A) and the compound (B) thus obtained generally contains more than 0.2 parts by mass of the compound (B) with respect to 100 parts by mass of the compound (A).

(Purifying Step)

In the present embodiment, the mixture containing the compound (A) and the compound (B) thus obtained is purified by a distillation process at a reflux ratio (reflux/distillate) of 2.0 to 4.0, a pressure of 1.0 to 10.0 kPa, and a distillation temperature of 90 to 140°C, and the compound (A) is recovered as a low-boiling component. As a result, a purified product containing 0.00002 to 0.2 parts by mass of the compound (B) with respect to 100 parts by mass of the compound (A) are obtained. Since the compound (A) is usually a liquid, no solvent is required.

In the present embodiment, since the mixture is purified by the distillation process at the reflux ratio of 2.0 to 4.0, the compound (B) can be efficiently removed. When the reflux ratio is less than 2.0, since the physical properties of the compound (A) and the compound (B) are similar, the compound (B) cannot be sufficiently removed, and the content of the compound (B) with respect to 100 parts by mass of the compound (A) does not fall below 0.2 parts by mass. The reflux ratio is preferably 2.5 or more in order to further reduce the content of the compound (B). When the reflux ratio is 4.0 or less, the purification step can be carried out efficiently in a short time, the yield of the compound (A) in the composition can be sufficiently ensured, and the composition can be produced in a high yield. The reflux ratio is preferably 3.5 or less in order to perform the purification step more efficiently and further improve the yield of the compound (A).

In this embodiment, the distillation temperature in the purification step is 90°C to 140°C. When the distillation temperature is less than 90°C, the compound (A) and the compound (B) cannot be sufficiently separated, and the content of the compound (B) with respect to 100 parts by mass of the compound (A) does not become 0.2 parts by mass or less. Further, when the distillation temperature is 140°C or lower, the compound (A) is not lost more than necessary, the yield of the compound (A) can be ensured, and the purifying step can be efficiently performed. In order to sufficiently remove compound (B) and to improve the yield of compound (A), the distillation temperature is preferably from 100°C to 130°C, and more preferably from 110°C to 120°C.

In this embodiment, the pressure at the time of distillation in the purification step is 1.0 to 10.0 kPa, and preferably 1.0 to 6.0 kPa. When the pressure is 1.0 kPa or more, a flooding phenomenon is unlikely generated at a distillation temperature of 90 to 140°C, and a stable distillation state is easily maintained. When the pressure is 10.0 kPa or less, the compound (A) and the compound (B) are easily separated at a distillation temperature of 140°C or less, and the loss of the compound (A) due to increasing the distillation temperature is preferably suppressed.

When the distillation process is performed in the purifying step, a polymerization inhibitor may be added to the mixture before heating of the mixture is started. By adding the polymerization inhibitor to the mixture before starting the heating of the mixture, the mixture is prevented from gelation by polymerization due to the temperature rise accompanying the distillation process.

The polymerization inhibitor added to the mixture is partially removed by the distillation process. The polymerization inhibitor remaining in the composition after the distillation process prevents the composition from gelation during storage and transportation of the composition and contributes to improvement of stability during storage of the composition. The polymerization inhibitor may optionally be added to the composition obtained after the distillation process.

Specific examples of polymerization inhibitors include hydroquinone, methoxyhydroquinone, catechol, p-tert-butylcatechol, cresol, 2,6-ditert-butyl-4-methylphenol (BHT), phenothiazine, 2,6-di-t-butyl-p-cresol and the like.

The purifying step includes a vacuum breaking step of supplying a gas having a dew point of -30°C or lower and containing nitrogen gas and oxygen gas at an oxygen concentration of 1 vol% or more and less than the critical oxygen concentration of the compound (A) into the distillation apparatus after the distillation process and returning the pressure in the distillation apparatus to the atmospheric pressure; and a filling step of storing the purified product in the distillation apparatus after the distillation process in a container and filling the gas phase portion in the container with air having a dew point of -30°C or lower.

The term "critical oxygen concentration" in this embodiment means the highest oxygen concentration (vol%) at which the compound (A) does not explode, which is obtained by examining the relationship between the oxygen concentration in the gaseous state contained in the compound (A) in the gaseous state and the presence or absence of explosion. For example, when the compound (A) is 2-methacryloyloxyethyl isocyanate (MOI), the critical oxygen concentration of the compound (A) is 8 vol%. When the compound (A) is 2-acryloyloxyethyl isocyanate (AOI), the critical oxygen concentration of the compound (A) is 7 vol%.

The presence of absence of explosion of gas composed of the compound (A) and oxygen, can be examined using Kitagawa explosion limit test equipment. Specifically, a sample, which is a gas composed of compound (A) and oxygen, is introduced into an explosion cylinder and is subjected to a test of spark discharge for one second in the explosion cylinder. In this test, when the cover of the explosion tube is blown by the spark discharge, it is judged as "explosion", and when the cover of the explosion tube is not blown by the spark discharge, it is judged as "non-explosion". If three tests are conducted and all the tests show "non-explosion", the explosion is regarded as "none".

The oxygen gas concentration in the gas supplied into the distillation apparatus in the vacuum breaking step can be measured, for example, by using a zirconia type oxygen concentration meter.

Under the condition in the distillation apparatus at the time of the vacuum breaking step, bonding between ethylenically unsaturated groups of the compound (A) is likely to occur. In the present embodiment, in the vacuum breaking step, a gas having a dew point of -30°C or lower containing nitrogen gas and oxygen gas at an oxygen concentration of 1 vol% or more and less than the critical oxygen concentration of the compound (A) is supplied into the distillation apparatus after distillation to return the pressure in the distillation apparatus to the atmospheric pressure, so that the bonding between the ethylenically unsaturated groups of the compound (A) in the purified product after distillation is suppressed. Further, in the present embodiment, in the filling step, the gas phase portion in the container is filled with air having a dew point of -30°C or lower, so that the bonding between the ethylenically unsaturated groups of the compound (A) in the composition contained in the container is suppressed. Therefore, the formation or increase of the compound (B) in the composition can be prevented by performing the vacuum breaking step and the filling step. Therefore, the stability during storage and the stability during use are improved.

In the present embodiment, the oxygen concentration in the gas supplied into the distillation apparatus in the vacuum breaking step is 1 vol% or more so as to obtain a polymerization inhibiting effect by oxygen. In addition, the oxygen concentration in the gas supplied into the distillation apparatus in the vacuum breaking step is set below the critical oxygen concentration of the compound (A) so that the substance in the distillation apparatus does not ignite or explode. The oxygen concentration in the gas supplied into the distillation apparatus in the vacuum breaking step is preferably 1 to 6 vol%, more preferably 2 to 5 vol%, in order to more effectively suppress bonding between ethylenically unsaturated groups of the compound (A) in the purified product after distillation.

A gas obtained by diluting air with nitrogen gas is preferably used as the gas containing nitrogen gas and oxygen gas at an oxygen concentration of 1 vol% or more and lower than the critical oxygen concentration of the compound (A).

The dew point of the gas supplied into the distillation apparatus in the vacuum breaking step and the air filled into the gas phase portion in the container in the filling step is preferably -30°C or lower and -40°C or lower in order to suppress the reaction between the compounds (A) in the purified product. The dew point of the gas to be supplied into the distillation apparatus in the vacuum breaking step and the air to be filled into the gas phase portion in the container in the filling step are preferably -70°C or higher because they are industrially readily available.

In the present embodiment, in order to more effectively prevent the increase of the compound (B) in the composition, it is preferable to store the purified product in the distillation apparatus after distillation in a container having air permeability.

[Method for Producing Unsaturated Compound]

The method of producing an unsaturated compound according to the present embodiment includes a step of mixing the composition with a compound having active hydrogen and reacting the compound (A) contained in the composition with the compound having active hydrogen to obtain a reaction product.

In the present embodiment, the compound (A) contained in the composition used as the material of the unsaturated compound can be appropriately selected according to the structure of the unsaturated compound.

The active hydrogen in the compound having the active hydrogen is a hydrogen atom bonded to a nitrogen atom, an oxygen atom, a sulfur atom or the like, and exhibits high reactivity as compared with a hydrogen atom bonded to a carbon atom. The compound having active hydrogen is not particularly limited, and can be appropriately selected according to the structure of the unsaturated compound.

For example, when a compound having an active hydrogen-containing group, such as a hydroxyl group, a mercapto group, an amino group (including cyclic amines, amides and imides), and a carboxy group, is used as the active hydrogen-containing compound, the following reaction product (unsaturated compound) is obtained by the following reaction.

When a compound (A) contained in the composition is reacted with a compound having a hydroxyl group, the isocyanate group of the compound (A) reacts with the hydroxyl group to form an unsaturated urethane compound. In this embodiment, the unsaturated urethane compound means a compound containing an ethylenically unsaturated bond and a urethane bond in the molecule.

When a compound (A) contained in the composition is reacted with a compound having a mercapto group, the isocyanato group of the compound (A) is reacted with the mercapto group to form an unsaturated thiourethane compound. In this embodiment, the unsaturated thiourethane compound means a compound containing an ethylenically unsaturated bond and a thiourethane bond in the molecule.

When a compound (A) contained in the composition is reacted with a compound having an amino group, the isocyanate group of the compound (A) is reacted with the amino group to form an unsaturated urea compound. In this embodiment, the unsaturated urea compound means a compound containing an ethylenically unsaturated bond and a urea bond in the molecule.

When a compound (A) contained in the composition is reacted with a compound having a carboxy group, the isocyanato group of the compound (A) is reacted with the carboxy group to form an unsaturated amide compound. In this embodiment, the unsaturated amide compound means a compound containing an ethylenically unsaturated bond and an amide bond in the molecule.

The compound having a hydroxyl group can be arbitrarily selected, examples thereof include an aliphatic alcohol compound such as ethanol, n- or iso-propanol, butanol of its isomer, pentanol, hexanol, octanol, decanol, or the like; phenolic compounds such as phenol, cresol, p-nonylphenol, methyl salicylate; aliphatic polyols such as ethylene glycol, diethylene glycol, propylene glycol, tetramethylenediol, neopentyl glycol, 1,6-hexanediol, glycerol, trimethylolethane, trimethylolpropane, butanetriol, pentaerythritol, dipentaerythritol, tripentaerythritol, sorbitol, hexanetriol, triglycerol, polyethylene glycol, polypropylene glycol, ethylene oxide and propylene oxide copolymers, tris(2-hydroxyethyl) isocyanurate, cyclohexanediol, cyclohexanedimethanol, hydroxypropylhexanol, tricyclo [5, 2, 3, 0$^{2, 6}$] decanedimethanol, dicyclohexanediol, or the like; aromatic polyols such as dihydroxynaphthalene, dihydroxybenzene, bisphenol-A, bisphenol-F, pyrogallol, xylene glycol, bisphenol-A (2-hydroxyethyl ether); halogenated polyols such as dibromoneopentyl glycol; hydroxyl group-containing epoxy resin; phenoxy resin; polymeric polyols such as polyvinyl alcohol and (co) polymers of hydroxyethyl (meta) acrylate; terminal hydroxyl-containing reaction product obtained by reacting the above-mentioned polyol with an organic acid such as phthalic acid, pyromellitic acid, trimellitic acid, adipic acid, dimer acid or the like; addition reaction product of the above-mentioned polyols and alkylene oxide (ethylene oxide, propylene oxide, and the like); glucose derivatives such as hydroxyethyl cellulose and nitrocellulose; heterocycle-containing alcohols such as the carboxylic acid (formic acid, acetic acid, benzoic acid, or the like) orthoester of pentaerythritol; groups with both hydrogen atom and mercapto group, such as 2-mercaptoethanol; oxime-based compounds such as dimethyl ketone oxime, diethyl ketone oxime, methyl ethyl ketone oxime (MEK oxime) or the like; and the like.

Among the compounds having a hydroxyl group, polyol is preferable, and aliphatic polyol is more preferable.

The compound having the mercapto group can be optionally selected, and examples thereof include monothiols such as 1-butanethiol, 1-pentanethiol, 1-octanethiol, 1-dodecanethiol, n-octadecanethiol, $\alpha$-toluenethiol, 2-benzimidazolethiol, 2-thiazoline-2-thiol, 2-methyl-2-propunchol, o-aminothiophenol, or the like; hexanedithiol, decanedithiol, 1,4-butanediol bisthiopropionate, 1,4-butanediol bisthioglycolate, ethylene glycol bisthioglycolate, ethylene glycol bisthioglycolate, ethylene glycol bisthiopropionate, trimethylolpropane trithioglycolate, trimethylolpropane tristhiopropionate, trimethylolpropane tristhiopropionate (3-mercaptobutylate), pentaerythritol tetrakis (2-mercaptopropionate), trimercaptopropionate tris (2-hydroxyethyl) isocyanurate, 1,4-dimethylmercaptobenzene, 2,4,6-trimercapto-s-triazine, 2- (N, N-dibutylamino)-4, 6-dimercapto-s-triazine, tetraethylene glycol bis (3-mercaptopropionate), trimethylolpropane tris (3-mercaptopropionate), tris (3-mercaptopropionyloxyethyl) isocyanurate, pentaerythritol tetrakis 3-mercaptopropionate, aliphatic polythiols, such as dipentaerythritol tetrakis (3-mercaptopropionate), 1,4-bis (3-mercaptobutyryloxy) butane (examples of specific products: "KarenzMT (registered trademark) BD1"), 1,3,5-tris (3-mercaptobutylate)-1,3,5-triazine-2,4,6 (1H, 3H, 5H)-trione (examples of specific products: "KarenzMT(registered trademark) NR1"), pentaerythritol tetrakis (3-mercaptobutylate) (examples of specific products: "KarenzMT (registered trademark) PE1"); and the like.

Among the compounds having a mercapto group, polythiol is preferable, and aliphatic polythiol is more preferable.

The compound having an amino group can be arbitrarily selected, but examples thereof include a monoamine such as butylamine, hexylamine or aniline; aliphatic polyamines such as diethylenetriamine, triethylenetetramine, 1,3- of 1,4-bisaminomethylcyclohexane, isophoronediamine, hexamethylenediamine, bis(4-aminocyclohexyl) methane or the like; aromatic polyamines such as m- or p-xylylenediamine, bis (4-aminophenyl) methane, 2,4- or 2,6-tolylenediamine or the like; glucosamines such as chitosan or the like; silicone compounds such as bis(3-aminopropyl) polydimethylsiloxane and bis (3-aminopropyl) polydiphenylsiloxane; heterocycle-containing compounds such as imidazole, $\varepsilon$-caprolactam, phthalimide or the like; amides; imides; 2-[(3,5-dimethylpyrazolyl) carbonylamino] ethyl methacrylate (examples of specific products: "KarenzMOI-BP (registered trademark)"); 2-[(3,5-dimethylpyrazolyl) carbonylamino] ethyl acrylate; 3,5-dimethylpyrazole; and the like.

Among the compounds having an amino group, polyamines are preferable, and aliphatic polyamines are more preferable.

The compound having a carboxy group can be arbitrarily selected, but a monocarboxylic acid such as acetic acid, propionic acid, decanoic acid or the like; aliphatic and aromatic polycarboxylic acids such as succinic acid, adipic acid, phthalic acid, isophthalic acid, terephthalic acid, trimellitic acid, pyromellitic acid or the like; polymeric polycarboxylic acids such as polyamic acids, (co) polymers of acrylic acids or the like can be used.

Among the compounds having a carboxy group, polycarboxylic acids are preferable, and aliphatic and aromatic polycarboxylic acids are more preferable.

Further, as the compound having the active hydrogen, a halogen substitution such as a fluorine substitution or a chlorine substitution of the compound having the active hydrogen may be used. These may be used alone or in combination of two or more.

The active hydrogen-containing compound is preferably a polyol, a polythiol, a polyamine or a polycarboxylic acid in view of versatility, and is particularly preferably a polyol.

In the reaction of the compound (A) contained in the composition of the present embodiment with the compound having active hydrogen, the ratio of the compound (A) to the compound having active hydrogen is set in consideration of the ratio of isocyanate groups/active hydrogen.

The isocyanato group/active hydrogen ratio may be the same as that conventionally applied in the reaction of compound (A) with a compound having active hydrogen. The ratio of isocyanato group to active hydrogen varies depending on the kind of compound having active hydrogen.

The compound (A) contained in the composition of the present embodiment and the compound having active hydrogen may be reacted in the presence of a reaction catalyst. The reaction rate can be controlled by the addition amount of the reaction catalyst.

As the reaction catalyst, a known reaction catalyst can be used. Specific examples of reaction catalysts include dibutyltin dilaurate, copper naphthenate, cobalt naphthenate, zinc naphthenate, triethylamine, 1,4-diazabicyclo [2.2.2] octane, zirconium acetylacetonate, titanium diisopropoxybis (ethyl acetoacetate), mixtures of bismuth tris(2-ethylhexanoate) and 2-ethylhexanoic acid, and the like. These reaction catalysts may be used singly or in combination of two or more.

When the compound (A) contained in the composition of the present embodiment is reacted with a compound having active hydrogen, the reaction temperature is preferably - 10 to 100°C, and more preferably 0 to 80°C.

When the compound (A) contained in the composition of the present embodiment is reacted with a compound having active hydrogen, a polymerization inhibitor may be added if necessary. As the polymerization inhibitor, a generally used one can be used, for example, a phenolic compound, a hydroquinone compound or the like can be used. Specific examples of polymerization inhibitors include hydroquinone, methoxyhydroquinone, catechol, p-tert-butylcatechol, cresol, 2,6-di-tert-butyl-4-methylphenol (BHT), and the like.

In addition, various substances such as known light stabilizers, ultraviolet absorbers, antioxidants, dye fillers, reactive diluents and the like may be added according to the purpose of the reaction.

The unsaturated compound (reaction product) is preferably at least one selected from the group consisting of unsaturated urethane compounds, unsaturated thiourethane compounds, unsaturated urea compounds and unsaturated amide compounds, and more preferably at least one selected from the group consisting of 2-butanone oxime-O-(carbamoylethyl-2-methacrylate), 2-[(3,5-dimethylpyrazolyl)carbonylamino]ethyl methacrylate, 2-butanone oxime-O-(carbamoylethyl-2-acrylate) and 2-[(3,5-dimethylpyrazolyl)carbonylamino] ethyl acrylate.

The unsaturated compound thus obtained is preferably used as a material in various fields such as coating materials, adhesives, photoresists, contact lenses, solid electrolytes, solidification of bioactive substances or the like.

The composition of the present embodiment is a composition which contains a compound (A) represented by general formula (1) and a compound (B) in which 2 or more molecules of the compound (A) are oligomers formed by bonding between ethylenically unsaturated groups of each compound (A) and which contains 0.00002 to 0.2 parts by mass of the compound (B) with respect to 100 parts by mass of the compound (A). Therefore, it is excellent in stability during storage and stability during use. Further, it is not necessary to contain a large amount of the polymerization inhibitor which forms the colored component. Therefore, it is possible to prevent the unsaturated compound produced by using the composition of the present embodiment from being colored by the coloring component caused by the polymerization inhibitor. Further, since the composition of the present embodiment contains 0.00002 parts by mass or more of the compound (B) with respect to 100 parts by mass of the compound (A), the composition can be produced in a high yield.

In the method of producing the composition of the present embodiment, a mixture, which contains more than 0.2 parts by mass of the compound (B) with respect to 100 parts by mass of the compound (A), is purified by a distillation process at a reflux ratio of 2.0 to 4.0, a pressure of 1.0 to 10.0 kPa, and a distillation temperature of 90 to 140°C to obtain a purified product containing 0.00002 to 0.2 parts by mass of the compound (B) with respect to 100 parts by mass of the compound (A). Therefore, according to the method of producing the composition of the present embodiment, the composition of the present embodiment can be produced in a high yield in which the compound (B) that affects the stability during storage and affects the stability during use is sufficiently removed from the mixture.

Further, in the method of producing the composition of the present embodiment, in the purification step, a vacuum breaking step of supplying a gas having a dew point of - 30°C or lower containing nitrogen gas and oxygen gas having an oxygen concentration of 1 vol% or more and less than the critical oxygen concentration of the compound (A) into the distillation apparatus after distillation to return the pressure in the distillation apparatus to the atmospheric pressure is performed; and a filling step of storing the purified product in the distillation apparatus after distillation into a container and filling the gas phase portion in the container with air having a dew point of -30°C or lower is performed. Therefore, the ethylenically unsaturated groups of the compound (A) unlikely bond to each other in the purified product after distillation, and the formation or increase of the compound (B) in the purified product can be prevented. Therefore, according to the method of producing the composition of the present embodiment, a composition having good stability during storage and stability during use can be obtained.

The method of producing the unsaturated compound of the present embodiment includes a step of mixing the composition of the present invention with a compound having active hydrogen and reacting the compound (A) contained in the composition with the compound having active hydrogen to obtain a reaction product. In the method of producing the unsaturated compound according to the present embodiment, since the composition used as the material contains 0.00002 to 0.2 parts by mass of the compound (B) with respect to 100 parts by mass of the compound (A), rapid increase in viscosity or gelation of the reaction product is unlikely to occur during production, and excellent productivity is obtained.

EXAMPLES

The present invention will be specifically described below with reference to examples and comparative examples. The following examples are intended to facilitate understanding of the contents of the present invention.

For example, in all examples, the content of compound (A) in the composition is 95.0% by mass or more. The content of the compound (A) in the composition may be, for example, less than 95.0% by mass.

Mixture 1 and Mixture 2 were prepared by the following methods.

<Mixture 1> (Synthesis of MOI)

In a 500 ml four-necked flask equipped with a stirrer, condenser, thermometer and inner tube, 250 ml of toluene and 25 g (0.41 mol) of 2-aminoethanol were added, heated to 90°C and supplied with about 20 g of hydrogen chloride gas. Then, 44 g (0.42 mol) of methacrylic acid chloride was added dropwise, and the mixture was heated at 90°C for 1 hour. Thereafter, 80 g (0.81 mol) of phosgene was supplied. 0.4 g of phenothiazine and 0.4 g of 2,6-bis-tert-butylhydroxytoluene were then added to remove dissolved phosgene and toluene.

By the above process, Mixture 1 containing the main product (the compound (A)) of 2-methacryloyloxyethyl isocyanate (MOI) 45 g (0.29 mol) (71% yield) and the by-product (the compound (B)) of 2-methacryloyloxyethyl isocyanate oligomer (Oligomer formed by reaction of double bond) (18034 ppm by mass) was obtained.

<Mixture 2> (Synthesis of AOI)

In a 500 mL four-necked flask equipped with a stirrer, condenser, thermometer and inner tube, 250 mL of toluene and 25 g (0.41 mol) of 2-aminoethanol were added, heated to 90°C and supplied with about 20 g of hydrogen chloride gas. Then 56 g (0.44 mol) of 3-chloropropionic acid chloride was added dropwise over 90 minutes and heated at 90°C for 1 hour. Thereafter, 80 g (0.81 mol) of phosgene was supplied. Dissolved phosgene was then removed by nitrogen gas bubbling. Subsequently, 0.4 g of phenothiazine and 0.4 g of 2,6-bis-t-butylhydroxytoluene were added, and 50 g of triethylamine (0.49 mol) was supplied, and the mixture was heated and stirred at 50°C for 6 hours. Thereafter, the mixture was cooled to room temperature, the formed hydrochloride salt was filtered, and toluene was distilled off.

By the above process, Mixture 2 containing the main product (the compound (A)) of 2-acryloyloxyethyl isocyanate (AOI) 55 g (0.35 mol) (87% yield) and the by-product (the compound (B)) of 2-acryloyloxyethyl isocyanate oligomer (Oligomer formed by reaction of double bond) (27530 ppm by mass) was obtained.

<Examples 1 to 6 and Comparative Examples 1 to 8> (MOI)

50 g of Mixture 1 was distilled under the conditions shown in Tables 1 and 2 (reflux ratio (reflux/distillate), distillation temperature, distillation pressure). The liquid compositions of Examples 1 to 6 and Comparative Examples 1 to 8 housed in a container were obtained by carrying out a vacuum breaking step in which the gas shown in Tables 1 and 2 was supplied into the distillation apparatus after distillation to return the pressure in the distillation apparatus to atmospheric pressure; and a filling step in which the purified product in the distillation apparatus after distillation was housed in the containers shown in Tables 1 and 2 and the gas phase portion in the container was filled with the gas shown in Tables 1 and 2.

The critical oxygen concentration of 2-methacryloyloxyethyl isocyanate (MOI) is 8 vol%. The oxygen gas concentration in the gas supplied into the distillation apparatus in the vacuum breaking step was measured using a zirconia type oxygen concentration meter.

<Examples 7 to 12 and Comparative Examples 9 to 16> (AOI)

50 g of Mixture 2 was distilled under the conditions shown in Tables 3 and 4 (reflux ratio (reflux/distillate), distillation temperature, distillation pressure). The liquid composition of Examples 7 to 12 and Comparative Examples 9 to 16 accommodated in a container was obtained by carrying out a vacuum breaking step in which the gas shown in Tables 3 and 4 was supplied into the distillation apparatus after distillation to return the pressure in the distillation apparatus to atmospheric pressure; and a filling step in which the purified product in the distillation apparatus after distillation was accommodated in the containers shown in Tables 1 and 2 and the gas phase portion in the container was filled with the gas shown in Tables 3 and 4.

The critical oxygen concentration of 2-acryloyloxyethyl isocyanate (AOI) is 7 vol%. The oxygen gas concentration in the gas supplied into the distillation apparatus in the vacuum breaking step was measured using a zirconia type oxygen concentration meter.

Table 1]

| Example | 1 | 2 | 3 | 4 | 5 | 6 |
|---|---|---|---|---|---|---|
| Distillation temperature (°C) | 110 | 115 | 90 | 100 | 135 | 130 |

(continued)

| Example | 1 | 2 | 3 | 4 | 5 | 6 |
|---|---|---|---|---|---|---|
| Distillation pressure (kPa) | 2.0 | 2.5 | 1.0 | 1.5 | 6.0 | 6.0 |
| Reflux ratio | 3.5 | 3.0 | 3.5 | 2.5 | 2.0 | 4.0 |
| Gas supplied in the vacuum breaking step | Dried nitrogen Dilution air (Dew point: -40°C) (Oxygen: 3 vol%) | Dried nitrogen Dilution air (Dew point: -40°C) (Oxygen: 4 vol%) | Dried nitrogen Dilution air (Dew point: -40°C) (Oxygen: 3 vol%) | Dried nitrogen Dilution air (Dew point: -50°C) (Oxygen: 4 vol%) | Dried nitrogen Dilution air (Dew point: -40°C) (Oxygen: 6 vol%) | Dried nitrogen Dilution air (Dew point: -30°C) (Oxygen: 5 vol%) |
| Gas filled in the filling step | Dried air (Dew point: -50°C) | Dried air (Dew point: -60°C) | Dried air (Dew point: -30°C) | Dried air (Dew point: -30°C) | Dried air (Dew point: -50°C) | Dried air (Dew point: -60°C) |
| Container | Chemical drum with high-density PE inner bag | Chemical drum with high-density PE inner bag | Chemical drum with high-density PE inner bag | Chemical drum with high-density PE inner bag | Chemical drum with high-density PE inner bag | Chemical drum with high-density PE inner bag |
| Compound A content (% by mass) | 98.0 | 98.5 | 98.7 | 98.2 | 99.2 | 99.4 |
| Compound B Content ($\times 10^{-4}$ parts by mass) | 540 | 150 | 204 | 560 | 360 | 450 |
| Yield (%) | 85 | 87 | 90 | 88 | 84 | 78 |
| Viscosity (mPa·sec) | 1.5 | 1.7 | 1.6 | 1.7 | 1.7 | 1.6 |
| Appearance | No change | No change | No change | No change | No change | No change |

[Table 2]

| Comparative Example | 1 | 2 | 3 | 4 | 5 | 6 | 7 | 8 |
|---|---|---|---|---|---|---|---|---|
| Distillation temperature (°C) | 90 | 110 | 135 | 110 | 90 | 100 | 135 | 130 |
| Distillation pressure (kPa) | 1.0 | 2.0 | 6.5 | 2.0 | 1.0 | 1.5 | 6.0 | 6.0 |
| Reflux ratio | 1.0 | 0.2 | 1.5 | 0.5 | 3.5 | 2.5 | 2.0 | 4.5 |
| Gas supplied in the vacuum breaking step | Dried nitrogen Dilution air (Dew point: -40°C) (Oxygen: 3 vol%) | Dried nitrogen Dilution air (Dew point: -50°C) (Oxygen: 4 vol%) | Dried nitrogen Dilution air (Dew point: -50°C) (Oxygen: 6 vol%) | Dried nitrogen Dilution air (Dew point: -60°C) (Oxygen: 5 vol%) | Dried nitrogen (Dew point: -30°C) | Dried nitrogen (Dew point: -50°C) | Dried nitrogen Dilution air (Dew point: -40°C) (Oxygen: 4 vol%) | Dried nitrogen (Dew point: -30°C) |
| Gas filled in the filling step | Dried air (Dew point: -50°C) | Dried air (Dew point: -60°C) | Dried air (Dew point: -40°C) | Dried air (Dew point: -40°C) | Dried nitrogen (Dew point: -40°C) | Dried air (Dew point: -30°C) | Dried nitrogen (Dew point: -50°C) | Dried nitrogen (Dew point: -60°C) |
| Container | Chemical drum with high-density PE inner bag | Chemical drum with high-density PE inner bag | Chemical drum with high-density PE inner bag | Chemical drum with high-density PE inner bag | Chemical drum with high-density PE inner bag | Chemical drum with high-density PE inner bag | Chemical drum with high-density PE inner bag | Chemical drum with high-density PE inner bag |
| Compound A content (% by mass) | 97.5 | 97 | 97.8 | 96.3 | 98.2 | 97.7 | 98.7 | 98.9 |
| Compound B Content ($\times 10^{-4}$ parts by mass) | 2200 | 2650 | 3302 | 2813 | 4650 | 3362 | 4870 | 6897 |
| Yield (%) | 93 | 90 | 85 | 88 | 90 | 88 | 84 | 78 |
| Viscosity (mPa-sec) | Not measurable | Not measurable | Not measurable | Not measurable | Not measurable | Not measurable | Not measurable | Not measurable |
| Appearance | Syrup-like | Solid | Syrup-like | Solid | Syrup-like | Solid | Syrup-like | Solid |

EP 3 842 416 B1

14

[Table 3]

| Example | 7 | 8 | 9 | 10 | 11 | 12 |
|---|---|---|---|---|---|---|
| Distillation temperature (°C) | 90 | 120 | 130 | 110 | 115 | 125 |
| Distillation pressure (kPa) | 1.5 | 3.5 | 6.5 | 2.5 | 3.0 | 4.5 |
| Reflux ratio | 3.5 | 2.0 | 3.5 | 2.5 | 2.0 | 4.0 |
| Gas supplied in the vacuum breaking step | Dried nitrogen Dilution air (Dew point: -50°C) (Oxygen: 4 vol%) | Dried nitrogen Dilution air (Dew point: -60°C) (Oxygen: 3 vol%) | Dried nitrogen Dilution air (Dew point: -40°C) (Oxygen: 3 vol%) | Dried nitrogen Dilution air (Dew point: -30°C) (Oxygen: 4 vol%) | Dried nitrogen Dilution air (Dew point: -40°C) (Oxygen: 5 vol%) | Dried nitrogen Dilution air (Dew point: -40°C) (Oxygen: 6 vol%) |
| Gas filled in the filling step | Dried air (Dew point: -40°C) | Dried air (Dew point: -30°C) | Dried air (Dew point: -50°C) | Dried air (Dew point: -40°C) | Dried air (Dew point: -50°C) | Dried air (Dew point: -60°C) |
| Container | Chemical drum with high-density PE inner bag | Chemical drum with high-density PE inner bag | Chemical drum with high-density PE inner bag | Chemical drum with high-density PE inner bag | Chemical drum with high-density PE inner bag | Chemical drum with high-density PE inner bag |
| Compound A content (% by mass) | 98.6 | 98.5 | 99.4 | 98.2 | 99.2 | 99.6 |
| Compound B Content (x$10^{-4}$ parts by mass) | 542 | 1203 | 658 | 897 | 1567 | 795 |
| Yield (%) | 88 | 82 | 80 | 86 | 84 | 72 |
| Viscosity (mPa·sec) | 1.6 | 1.8 | 1.6 | 1.7 | 1.7 | 1.6 |
| Appearance | No change | No change | No change | No change | No change | No change |

[Table 4]

| Comparative Example | 9 | 10 | 11 | 12 | 13 | 14 | 15 | 16 |
|---|---|---|---|---|---|---|---|---|
| Distillation temperature (°C) | 90 | 110 | 130 | 110 | 130 | 110 | 115 | 125 |
| Distillation pressure (kPa) | 1.5 | 2.5 | 6.5 | 2.5 | 6.5 | 2.5 | 3.0 | 4.5 |
| Reflux ratio | 1.0 | 0.2 | 1.5 | 0.5 | 3.5 | 2.5 | 2.0 | 4.5 |
| Gas supplied in the vacuum breaking step | Dried nitrogen Dilution air (Dew point :-40°C) (Oxygen: 3 vol%) | Dried nitrogen Dilution air (Dew point: -50°C) (Oxygen: 4 vol%) | Dried nitrogen Dilution air (Dew point: -40°C) (Oxygen: 6 vol%) | Dried nitrogen Dilution air (Dew point: -60°C) (Oxygen: 5 vol%) | Dried nitrogen (Dew point: -50°C) | Dried nitrogen gas (Dew point: -40°C) | Dried nitrogen Dilution air (Dew point: -40°C) (Oxygen: 4 vol%) | Dried nitrogen (Dew point: -50°C) |
| Gas filled in the filling step | Dried air (Dew point: -50°C) | Dried air (Dew point: -30°C) | Dried air (Dew point: -40°C) | Dried air (Dew point: -40°C) | Dried nitrogen (Dew point: -40°C) | Dried air (Dew point: -50°C) | Dried nitrogen (Dew point: -50°C) | Dried nitrogen (Dew point: -60°C) |
| Container | Chemical drum with high-density PE inner bag | Chemical drum with high-density PE inner bag | Chemical drum with high-density PE inner bag | Chemical drum with high-density PE inner bag | Chemical drum with high-density PE inner bag | Chemical drum with high-density PE inner bag | Chemical drum with high-density PE inner bag | Chemical drum with high-density PE inner bag |
| Compound A content (% by mass) | 97.5 | 97 | 97.8 | 96.3 | 99.4 | 98.2 | 99.2 | 99.6 |
| Compound B Content ($\times 10^{-4}$ parts by mass) | 2680 | 3356 | 3865 | 2745 | 4557 | 3023 | 4754 | 5754 |
| Yield (%) | 91 | 90 | 81 | 88 | 80 | 86 | 84 | 72 |
| Viscosity (mPa·sec) | Not measurable | Not measurable | Not measurable | Not measurable | Not measurable | Not measurable | Not measurable | Not measurable |
| Appearance | Syrup-like | Solid | Syrup-like | Solid | Syrup-like | Solid | Syrup-like | Solid |

Next, regarding the compositions of Examples 1 to 12 and Comparative Examples 1 to 16, the compounds (A) and the compounds (B) in the compositions were determined quantitatively by the following method, and the contents (% by mass) of the compounds (A) in the compositions and the contents ($\times 10^{-4}$ parts by mass) of the compounds (B) with respect to100 parts by mass of the compounds (A) were determined. The results are shown in Tables 1 to 4.

<Quantification of Compound (A)>

The compositions were analyzed by gas chromatography (GC) under the following conditions.

Column: DB-1, Inlet Temperature: 300°C, Detection Temperature: 300°C
Column temperature: 50°C to 300°C at 10°C/min
Column flow rate: 1.4 ml/min
Split ratio: 1/50
Detector: FID

< Quantification of compound (B)>

The composition was analyzed by liquid chromatography (LC) according to an internal standard method under the following conditions.

Column: Shodex (registered trademark) KF-801 (Product name: Manufactured by Showa Denko K.K.) $\times$ 4
Column temperature: 40°C
Solvent: THF (tetrahydrofuran)
Flow rate: 0.8 ml/min
Detector: RI (differential refractometer)

The yields (distillation yield) of the compositions obtained from Mixtures 1 of Mixtures 2 of Examples 1 to 12 and Comparative Examples 1 to 16 were determined by the following formula. The results are shown in Tables 1 to 4.

$$\text{Yield} = (\text{Composition Mass/Theoretical Yield of Compound A}) \times 100\ (\%)$$

"Appearance Evaluation"

In Examples 1 to 12 and Comparative Examples 1 to 16, 100 g of the liquid compositions immediately after distillation were stored in a container in a sealed state at 25°C for 30 days, and the appearance after storage was evaluated by the following method.
The container containing the composition was tilted several times at an angle of about 45 degrees and evaluated visually using the following criteria. The results are shown in Tables 1 to 4.

"Criteria"

No change: flowing in less than 30 seconds after tilting the container
Syrup-like: flowing down in 30 seconds or more and less than 180 seconds after tilting a container
Solid: not flowing in more than 180 seconds after tilting the container

"Method of Measuring Viscosity"

The viscosities of the compositions of Examples 1 to 12 and Comparative Examples 1 to 16 stored at 25°C for 30 days in a sealed state were determined in accordance with JIS-Z 8803: 2011 by the following method. The results are shown in Tables 1 to 4.
The kinematic viscosity of each composition at 25°C (cm3/sec) was measured using an Uberode viscometer. In Examples 1 to 6 and Comparative Examples 1 to 8, the measured kinematic viscosity was multiplied by the density of the following KarenzMOI (registered trademark) (manufactured by Showa Denko) to calculate the viscosity (mPa · sec). In Examples 7 to 12 and Comparative Examples 9 to 16, the viscosity was calculated by multiplying the measured value of the kinematic viscosity by the density of the KarenzAOI (registered trademark) (manufactured by Showa Denko) shown below (mPa · sec).

(Density of the KarenzMOI (registered trademark)) 1.096 g/cm$^3$ (25°C)

(Density of KarenzAOI (registered trademark)) 1.133 g/cm$^3$ (25°C)

As shown in Tables 1 to 4, the compositions of Examples 1 to 12, which contain 95.0 parts by mass of the compound (A) and which contain 0.00002 to 0.2 parts by mass of the compound (B) with respect to 100 parts by mass of the compound (A), had a sufficiently low viscosity after storage at 25°C for 30 days, and the appearance evaluation were "No change".

On the other hand, in the compositions of Comparative Examples 1 to 16, which contain more than 0.2 parts by mass of the compound (B) with respect to 100 parts by mass of the compound (A), the viscosity which became too high by storing at 25°C for 30 days, and the viscosity could not be measured. In the composition of Comparative Examples 1 to 16, the appearance evaluation was "syrup-like" or "solid".

(Unsaturated Compound)

<Example 13> (Reaction Product of (Poly)ol with MOI)

In a 500 ml four-necked flask equipped with a stirrer, reflux condenser and thermometer, 165 g of polyethylene glycol (number-average molecular weight 660) and 77.5 g of the composition of Example 1 (The compound (A) is MOI.) were charged and reacted at 80°C for 5 hours to synthesize an unsaturated urethane compound 1.

<Comparative Example 17> (Reaction Product of (Poly)ol with MOI)

An unsaturated urethane compound 2 was synthesized in the same manner as in Example 13 except that the composition of Comparative Example 1 (The compound (A) is MOI.) was used in place of the composition of Example 1.

<Comparative Example 18> (Reaction Product of (Poly)ol with MOI)

An unsaturated urethane compound 3 was synthesized in the same manner as in Example 13 except that the composition of Comparative Example 7 (The compound (A) is MOI.) was used in place of the composition of Example 1.

<Example 14> (Reaction Product of (Poly)ol with AOI)

In a 500 ml four-necked flask equipped with a stirrer, reflux condenser and thermometer, 165 g of polyethylene glycol (Number average molecular weight 660) and 70.5 g of the composition of Example 9 (The compound (A) is AOI.) were charged and reacted at 80°C for 5 hours to synthesize an unsaturated urethane compound 4.

<Comparative Example 19> (Reaction Product of (Poly)ol with AOI)

An unsaturated urethane compound 5 was synthesized in the same manner as in Example 14 except that the composition of Comparative Example 12 (The compound (A) is AOI.) was used in place of the composition of Example 9.

<Comparative Example 20> (Reaction Product of (Poly)ol with AOI)

An unsaturated urethane compound 6 was synthesized in the same manner as in Example 14 except that the composition of Comparative Example 16 (The compound (A) is AOI.) was used in place of the composition of Example 9.

Viscosities of the reaction liquids containing the unsaturated urethane compounds 1 to 6, obtained in Examples 13 and 14 and Comparative Examples 17 to 20, were measured in accordance with JIS-Z 8803: 2011 at 25°C using a tuning fork type vibrating viscometer (SV type viscometer manufactured by A & D Co., Ltd. (SV-10 Type)). The results are shown in Tables 5 and 6.

[Table 5]

| | Unsaturated Urethane Compound | Composition | Compound B Content (x10$^{-4}$ parts by mass) | Viscosity (mPa·sec) |
|---|---|---|---|---|
| Example 13 | 1 | Example 1 | 540 | 0.16 |
| Comparative Example 17 | 2 | Comparative Example 1 | 2200 | Gelation |
| Comparative Example 18 | 3 | Comparative Example 7 | 4847 | Gelation |

[Table 6]

| | Unsaturated Urethane Compound | Composition | Compound B Content (x10$^{-4}$ parts by mass) | Viscosity (mPa·sec) |
|---|---|---|---|---|
| Example 14 | 4 | Example 9 | 658 | 0.16 |
| Comparative Example 19 | 5 | Comparative Example 12 | 2745 | Gelation |
| Comparative Example 20 | 6 | Comparative Example 16 | 6754 | Gelation |

As shown in Table 5, in Example 13 in which the unsaturated urethane compounds were prepared by using the composition containing 95.0% by mass or more of the compound (A) and containing 0.00002 to 0.2 parts by mass of the compound (B) with respect to 100 parts by mass of the compound (A), the unsaturated urethane compound 1 having an appropriate viscosity was obtained, and the unsaturated urethane compound could be produced without problem.

On the other hand, in Comparative Examples 17 and 18 in which the unsaturated urethane compounds were prepared by using the composition containing more than 0.2 parts by mass of the compound (B) with respect to 100 parts by mass of the compound (A), the unsaturated urethane compounds 2 and 3 were gelatinized during the preparation of the unsaturated urethane compounds 2 and 3, although there was no problem in handling in the raw material stage.

As shown in Table 6, in Example 14 in which the unsaturated urethane compounds were prepared by using a composition containing 95.0% by mass or more of the compound (A) and containing 0.00002 to 0.2 parts by mass of the compound (B) with respect to 100 parts by mass of the compound (A), the unsaturated urethane compound 4 having an appropriate viscosity was obtained, and the unsaturated urethane compound could be produced without any problem.

On the other hand, in Comparative Examples 19 and 20 in which the unsaturated urethane compounds were prepared by using the composition containing more than 0.2 parts by mass of the compound (B) with respect to 100 parts by mass of the compound (A), the viscosity of the unsaturated urethane compounds 5 and 6 was high, and a part of them gelled during the preparation.

<Example 15> (Reaction Product of (Poly)amine with MOI)

A 500 ml four-necked flask equipped with a stirrer, reflux condenser and thermometer was charged with 66.4 g of (2-[(3,5-dimethylpyrazolyl)carbonylamino]ethyl methacrylate) (KarenzMOI-BP (registered trademark) manufactured by Showa Denko) and 77.4 g of 3,5-dimethylpyrazole, and 122.6 g of the composition of Example 2 (The compound (A) is MOI.) was supplied while the temperature was maintained at 35°C, and then reacted for 2 hours to synthesize an unsaturated urea compound 1.

<Comparative Example 21> (Reaction Product of (Poly)amine with MOI)

An unsaturated urea compound 2 was synthesized in the same manner as in Example 15 except that the composition of Comparative Example 2 (The compound (A) is MOI.) was used in place of the composition of Example 2.

<Comparative Example 22> (Reaction Product of (Poly)amine with MOI)

An unsaturated urea compound 3 was synthesized in the same manner as in Example 15 except that the composition of Comparative Example 5 (The compound (A) is MOI.) was used in place of the composition of Example 2.

<Example 16> (Reaction Product of (Poly)amine with AOI)

In a 1000 ml four-necked flask equipped with a stirrer, reflux condenser and thermometer, 115.9 g of 3,5-dimethylpyrazole and 155.0 g of 2-acetoxy-1-methoxypropane were charged, 174.0 g of the composition of Example 8 (The compound (A) is AOI.) was fed while maintaining the temperature at 15°C, and then reacted for 30 minutes. Subsequently, 320.0 g of n-hexane was added and cooled to 0°C to crystallize the unsaturated urea compound 4. The obtained crystals were recovered by filtration, washed with n-hexane, and dried under reduced pressure to isolate an unsaturated urea compound 4.

<Comparative Example 23> (Reaction Product of (Poly)amine with AOI)

An unsaturated urea compound 5 was synthesized in the same manner as in Example 16 except that the composition of Comparative Example 10 (The compound (A) is AOI.) was used in place of the composition of Example 8.

<Comparative Example 24> (Reaction Product of (Poly)amine with AOI)

An unsaturated urea compound 6 was synthesized in the same manner as in Example 16 except that the composition of Comparative Example 15 (The compound (A) is AOI.) was used in place of the composition of Example 8.
Viscosities of the reaction liquids containing the unsaturated urea compounds 1 to 6 obtained in Examples 15 and 16 and Comparative Examples 21 to 24 (before addition of n-hexane) were measured in accordance with JIS-Z 8803: 2011 at 25°C using a tuning fork type vibration type viscometer (SV type viscometer manufactured by A & D Co., Ltd. (SV -10 Type)). The results are shown in Tables 7 and 8.

[Table 7]

| | Unsaturated Urea Compound | Composition | Compound B Content (x$10^{-4}$ parts by mass) | Viscosity (mPa·sec) |
|---|---|---|---|---|
| Example 15 | 1 | Example 2 | 150 | 0.18 |
| Comparative Example 21 | 2 | Comparative Example 2 | 2650 | Gelation |
| Comparative Example 22 | 3 | Comparative Example 5 | 4650 | Gelation |

[Table 8]

| | Unsaturated Urea Compound | Composition | Compound B Content (x$10^{-4}$ parts by mass) | Viscosity (mPa·sec) |
|---|---|---|---|---|
| Example 16 | 4 | Example 8 | 1203 | 0.22 |
| Comparative Example 23 | 5 | Comparative Example 10 | 3356 | Gelation |
| Comparative Example 24 | 6 | Comparative Example 15 | 4754 | Gelation |

As shown in Table 7, in Example 15 in which unsaturated urea compounds were prepared by using the composition containing 95.0% by mass or more of the compound (A) and containing 0.00002 to 0.2 parts by mass of the compound (B) with respect to 100 parts by mass of the compound (A), the unsaturated urea compound 1 having an appropriate viscosity was obtained, and the unsaturated urea compound was successfully prepared.
On the other hand, in Comparative Examples 21 and 22 in which unsaturated urea compounds were prepared by using the composition containing more than 0.2 parts by mass of the compound (B) with respect to 100 parts by mass of the compound (A), the unsaturated urea compounds 2 and 3 were gelled during the preparation, although there was no problem in handling at the stage of raw material.

As shown in Table 8, in Example 16 in which unsaturated urea compounds were prepared by using a composition containing 95.0% by mass or more of the compound (A) and containing 0.00002 to 0.2 parts by mass of the compound (B) with respect to 100 parts by mass of the compound (A), the unsaturated urea compound 4 having a proper viscosity was obtained, and the unsaturated urea compound was successfully prepared.

On the other hand, in Comparative Examples 23 and 24 in which unsaturated urea compounds were prepared by using the composition containing more than 0.2 parts by mass of the compound (B) with respect to 100 parts by mass of the compound (A), the unsaturated urea compounds 5 and 6 were gelled during the preparation, although there was no problem in handling at the stage of raw material.

<Example 17> (Reaction Product of (Poly)carboxylic Acid with MOI)

In a 500 ml four-necked flask equipped with a stirrer, reflux condenser and thermometer, 177.3 g of decanoic acid, 156.5 g of the composition of Example 3 (The compound (A) is MOI.) and 0.8 g of dibutyltin dilaurate were charged and reacted at 80°C for 12 hours to synthesize an unsaturated amide compound 1.

<Comparative Example 25> (Reaction Product of (Poly)carboxylic Acid with MOI)

An unsaturated amide compound 2 was synthesized in the same manner as in Example 17 except that the composition of Comparative Example 3 (The compound (A) is MOI.) was used in place of the composition of Example 3.

<Comparative Example 26> (Reaction Product of (Poly)carboxylic Acid with MOI)

An unsaturated amide compound 3 was synthesized in the same manner as in Example 17 except that the composition of Comparative Example 8 (The compound (A) is MOI.) was used in place of the composition of Example 3.

<Example 18> (Reaction Product of (Poly)carboxylic Acid with AOI)

In a 500 ml four-necked flask equipped with a stirrer, reflux condenser and thermometer, 177.3 g of decanoic acid, 142.4 g of the composition of Example 7 (The compound (A) is AOI.) and 0.8 g of dibutyltin dilaurate were charged and reacted at 80°C for 12 hours to synthesize an unsaturated amide compound 4.

<Comparative Example 27> (Reaction Product of (Poly)carboxylic Acid with AOI)

An unsaturated amide compound 5 was synthesized in the same manner as in Example 18 except that the composition of Comparative Example 11 (The compound (A) is AOI.) was used in place of the composition of Example 7.

<Comparative Example 28> (Reaction Product of (Poly)carboxylic Acid with AOI)

An unsaturated amide compound 6 was synthesized in the same manner as in Example 18 except that the composition of Comparative Example 14 (The compound (A) is AOI.) was used in place of the composition of Example 7.

Viscosities of the reaction liquids containing the unsaturated amide compounds 1 to 6 obtained in Examples 17 and 18 and Comparative Example 25 to 28 were measured in accordance with JIS-Z 8803: 2011 at 25°C using a tuning fork type vibrating viscometer (SV type viscometer manufactured by A & D Co., Ltd. (SV -10 Type)). The results are shown in Tables 9 and 10.

[Table 9]

| | Unsaturated Amide Compound | Composition | Compound B Content ($\times 10^{-4}$ parts by mass) | Viscosity (mPa·sec) |
|---|---|---|---|---|
| Example 17 | 1 | Example 3 | 204 | 0.77 |
| Comparative Example 25 | 2 | Comparative Example 3 | 3302 | Gelation |
| Comparative Example 26 | 3 | Comparative Example 8 | 6897 | Gelation |

[Table 10]

|  | Unsaturated Amide Compound | Composition | Compound B Content (x10$^{-4}$ parts by mass) | Viscosity (mPa·sec) |
|---|---|---|---|---|
| Example 18 | 4 | Example 7 | 542 | 0.22 |
| Comparative Example 27 | 5 | Comparative Example 1 | 3865 | Gelation |
| Comparative Example 28 | 6 | Comparative Example 14 | 3023 | Gelation |

As shown in Table 9, in Example 17 in which unsaturated amide compounds were prepared by using the composition containing 95.0% by mass or more of the compound (A) and containing 0.00002 to 0.2 parts by mass of the compound (B) with respect to 100 parts by mass of the compound (A), the unsaturated amide compound 1 having a proper viscosity was obtained, and the unsaturated amide compound could be prepared without any problem.

On the other hand, in Comparative Examples 25 and 26 in which unsaturated amide compounds were prepared by using the composition containing more than 0.2 parts by mass of the compound (B) with respect to 100 parts by mass of the compound (A), although there was no problem in handling in the stage of raw material, they gelled during the preparation of the unsaturated amide compounds 2 and 3.

As shown in Table 10, in Example 18 in which unsaturated amide compounds were prepared by using the composition containing 95.0% by mass or more of the compound (A) and containing 0.00002 to 0.2 parts by mass of the compound (B) with respect to 100 parts by mass of the compound (A), the unsaturated amide compound 4 having a proper viscosity was obtained, and the unsaturated amide compound was successfully prepared.

On the other hand, in Comparative Examples 27 and 28 in which unsaturated amide compounds were prepared by using the composition containing more than 0.2 parts by mass of the compound (B) with respect to 100 parts by mass of the compound (A), although there was no problem in handling in the stage of raw material, they gelled during the preparation of the unsaturated amide compounds 5 and 6.

<Example 19> (Reaction Product of (Poly)thiol with MOI)

In a 500 ml four-necked flask equipped with a stirrer, reflux condenser and thermometer, 177.3 g of 1-octanethiol and 184.3 g of the composition of Example 4 (The compound (A) is MOI.) were charged and reacted at 80°C for 24 hours to synthesize an unsaturated thiourethane compound 1.

<Comparative Example 29> (Reaction Product of (Poly)thiol with MOI)

An unsaturated thiourethane compound 2 was synthesized in the same manner as in Example 19 except that the composition of Comparative Example 4 (The compound (A) is MOI.) was used in place of the composition of Example 4.

<Comparative Example 30> (Reaction of (Poly)thiol with MOI)

An unsaturated thiourethane compound 3 was synthesized in the same manner as in Example 19 except that the composition of Comparative Example 6 (The compound (A) is MOI.) was used in place of the composition of Example 4.

<Example 20> (Reaction Product of (Poly)thiol with AOI)

In a 500 ml four-necked flask equipped with a stirrer, reflux condenser and thermometer, 177.3 g of 1-octanethiol and 167.7 g of the composition of Example 12 (The compound (A) is AOI.) were charged and reacted at 80°C for 24 hours to synthesize an unsaturated thiourethane compound 4.

<Comparative Example 31> (Reaction Product of (Poly)thiol with AOI)

An unsaturated thiourethane compound 5 was synthesized in the same manner as Example 20 except that the composition of Comparative Example 9 (The compound (A) is AOI.) was used in place of the composition of Example 12.

<Comparative Example 32> (Reaction Product of (Poly)thiol with AOI)

An unsaturated thiourethane compound 6 was synthesized in the same manner as in Example 20 except that the composition of Comparative Example 13 (The compound (A) is AOI.) was used in place of the composition of Example 12.

Viscosities of the reaction liquids containing the unsaturated thiourethane compounds 1 to 6 obtained in Examples 19 and 20 and Comparative Examples 29 to 32 were measured in accordance with JIS-Z 8803: 2011 at 25°C using a tuning fork type vibrating viscometer (SV type viscometer manufactured by A & D Co., Ltd. (SV -10 Type)). The results are shown in Tables 11 and 12.

[Table 11]

| | Unsaturated Thiourethane Compound | Composition | Compound B Content (x10$^{-4}$ parts by mass) | Viscosity (mPa·sec) |
|---|---|---|---|---|
| Example 19 | 1 | Example 4 | 560 | 0.87 |
| Comparative Example 29 | 2 | Comparative Example 4 | 2813 | Gelation |
| Comparative Example 30 | 3 | Comparative Example 6 | 3362 | Gelation |

[Table 12]

| | Unsaturated Thiourethane Compound | Composition | Compound B Content (xlO-4.) Weight Part | Viscosity (mPa·sec) |
|---|---|---|---|---|
| Example 20 | 4 | Example 12 | 795 | 0.54 |
| Comparative Example 31 | 5 | Comparative Example 9 | 2680 | Gelation |
| Comparative Example 32 | 6 | Comparative Example 13 | 4557 | Gelation |

As shown in Table 11, in Example 19 in which unsaturated thiourethane compound was prepared by using the composition containing 95.0% by mass or more of the compound (A) and containing 0.00002 to 0.2 parts by mass of the compound (B) with respect to 100 parts by mass of the compound (A), the unsaturated thiourethane compound 1 having an appropriate viscosity was obtained, and the unsaturated thiourethane compound was successfully prepared.

On the other hand, in Comparative Examples 29 and 30 in which unsaturated thiourethane compounds were prepared by using the compositions containing more than 0.2 parts by mass of the compound (B) with respect to 100 parts by mass of the compound (A), although there was no problem in handling in the stage of raw material, the compound was gelled during the preparation of the unsaturated thiourethane compounds 2 and 3.

As shown in Table 12, in Example 20 in which unsaturated thiourethane compound was prepared by using the composition containing 95.0% by mass or more of the compound (A) and containing 0.00002 to 0.2 parts by mass of the compound (B) with respect to 100 parts by mass of the compound (A), the unsaturated thiourethane compound 4 having an appropriate viscosity was obtained, and the unsaturated thiourethane compound was successfully prepared.

On the other hand, in Comparative Examples 31 and 32 in which unsaturated thiourethane compounds were prepared by using the compositions containing more than 0.2 parts by mass of the compound (B) with respect to 100 parts by mass of the compound (A), the unsaturated thiourethane compounds 5 and 6 were gelatinized during the preparation of the unsaturated thiourethane compounds 5 and 6, although there was no problem in handling in the raw material stage.

<Example 21> (Reaction Product of Oxime compound with MOI)

In a 500 ml four-necked flask equipped with a stirrer, reflux condenser and thermometer, 167.0 g of 2-butanone oxime (hereinafter also referred to as "MEK oxime") was charged, and while the temperature was maintained at 35°C, 293.1 g of the composition of Example 6 (The compound (A) is MOI.) was fed and allowed to react for 2 hours to synthesize MOI-BM (2-butanone oxime-O-(carbamoylethyl-2-methacrylate) as unsaturated butanone oxime compound 1. MOI-BM is a mixture of (2-butanone oxime-O-(E)-(carbamoylethyl-2-methacrylate) and 2-butanone oxime-O-(Z)-(carbamoylethyl-2-methacrylate).

<Comparative Example 33> (Reaction Product of Oxime compound with MOI)

An unsaturated butanone oxime compound 2 was synthesized in the same manner as in Example 21 except that the composition of Comparative Example 3 (The compound (A) is MOI.) was used in place of the composition of Example 6.

<Comparative Example 34> (Reaction Product of Oxime compound with MOI)

An unsaturated butanone oxime compound 3 was synthesized in the same manner as in Example 21 except that the composition of Comparative Example 7 (The compound (A) is MOI.) was used in place of the composition of Example 6.

<Example 22> (Reaction Product of Oxime compound with AOI)

To a 500 ml four-necked flask equipped with a stirrer, reflux condenser and thermometer, at 15°C, 167.0 g of 2-butanone oxime (MEK oxime) and 266.7 g of the composition of Example 10 (The compound (A) is AOI.) were fed at the same time and reacted for 1 hour to synthesize AOI-BM (2-butanone oxime-O-(carbamoylethyl-2-acrylate) as an unsaturated butanone oxime compound 4. AOI-BM is a mixture of 2-butanone oxime-O-(E)-(Carbamoylethyl-2-acrylate) and 2-butanone oxime-O-(Z)-(carbamoylethyl-2-acrylate).

<Comparative Example 35> (Reaction Product of Oxime compound with AOI)

An unsaturated butanone oxime compound 5 was synthesized in the same manner as in Example 22 except that the composition of Comparative Example 11 (The compound (A) is AOI.) was used in place of the composition of Example 10.

<Comparative Example 36> (Reaction Product of Oxime compound with AOI)

An unsaturated butanone oxime compound 6 was synthesized in the same manner as in Example 22 except that composition of Comparative Example 15 (The compound (A) is AOI.) was used in place of the composition of Example 10.

The viscosities of the reaction liquids containing the unsaturated butanone oxime compounds 1 to 6, obtained in Examples 21 and 22 and Comparative Examples 33 to 36, were measured in accordance with JIS-Z 8803: 2011 at 25°C using a tuning-fork-type vibrating viscometer (SV type viscometer manufactured by A & D Co., Ltd. (SV-10 Type)). The results are shown in Tables 13 and 14.

[Table 13]

| | Unsaturated Butanone Oxime Compound | Composition | Compound B Content ($\times 10^{-4}$ parts by mass) | Viscosity (mPa·sec) |
|---|---|---|---|---|
| Example 21 | 1 | Example 6 | 450 | 0.15 |
| Comparative Example 33 | 2 | Comparative Example 3 | 3302 | Gelation |
| Comparative Example 34 | 3 | Comparative Example 7 | 4870 | Gelation |

[Table 14]

|  | Unsaturated Butanone Oxime Compound | Composition | Compound B Content (x10$^{-4}$ parts by mass) | Viscosity (mPa·sec) |
|---|---|---|---|---|
| Example 22 | 4 | Example 10 | 897 | 0.22 |
| Comparative Example 35 | 5 | Comparative Example 11 | 3865 | Gelation |
| Comparative Example 36 | 6 | Comparative Example 15 | 4754 | Gelation |

As shown in Table 13, in Example 21, in which unsaturated butanone oxime compounds were prepared by using compositions which contain 95.0 parts by mass of the compound (A) and which contain 0.00002 to 2.0 parts by mass of the compound (B) with respect to 100 parts by mass of the compound (A), the unsaturated butanone oxime compound 1 having proper viscosity were obtained, and the unsaturated butanone oxime compounds were successfully prepared.

On the other hand, in Comparative Examples 33 and 34 in which an unsaturated butanone oxime compound was prepared by using a composition which contains more than 0.2 parts by mass of the compound (B) with respect to 100 parts by mass of the compound (A), although there was no problem in handling in the stage of raw material, the compound gelled during the preparation of the unsaturated butanone oxime compounds 2 and 3.

As shown in Table 14, in Example 22, in which unsaturated butanone oxime compounds were prepared by using compositions which contain 95.0 parts by mass of the compound (A) and which contain 0.00002 to 0.2 parts by mass of the compound (B) with respect to 100 parts by mass of the compound (A), the unsaturated butanone oxime compound 4 having the proper viscosity were obtained, and the unsaturated butanone oxime compounds were successfully prepared.

On the other hand, in Comparative Examples 35 and 36, in which an unsaturated butanone oxime compound was prepared by using a composition which contains more than 0.2 parts by mass of the compound (B) with respect to 100 parts by mass of the compound (A), although there was no problem in handling in the stage of raw material, the compound gelled during the preparation of the unsaturated butanone oxime compounds 5 and 6.

As shown in the above results, depending on whether or not the content of the compound (B) with respect to 100 parts by mass of the compound (A) was 0.00002 to 0.2 parts by mass, there was a large difference in the behavior of the composition during storage. The viscosity of the unsaturated compound obtained by reacting the composition with any one of (poly)ol, (poly)amine, (poly)carboxylic acid, (poly)thiol and oxime compound also differed greatly depending on whether or not the content of the compound (B) with respect to 100 parts by mass of the compound (A) in the composition was 0.00002 to 0.2 parts by mass.

From these results, it was confirmed that the concentration of compound (B) in the composition is useful as an index for determining the stability of the composition during storage and as an index for determining whether or not a rapid increase in viscosity and/or gelation occurs during production when the unsaturated compound is produced by using the composition as a raw material.

**Claims**

1.   A composition comprising:

a compound (A) represented by general formula (1) and
a compound (B)
wherein the compound (B) is an oligomer in which two or more molecules of the compounds (A) are bonded to each other by an ethylenically unsaturated group of each compound (A); and
the composition comprises 0.00002 to 0.2 parts by mass of the compound (B) with respect to 100 parts by mass of the compound (A),

$$(R_1\text{-}COO)_n\text{-}R_2\text{-}(NCO)_m \qquad (1)$$

wherein in the general formula (1),
$R_1$ is an ethylenically unsaturated group having 2 to 7 carbon atoms;

$R_2$ is a (m + n)-valent hydrocarbon group having 1 to 7 carbon atoms and optionally comprises an ether group; and n and m each represent an integer of 1 or 2.

2. The composition according to claim 1, wherein the compound (A) is at least one compound selected from the group consisting of 2-methacryloyloxyethyl isocyanate, 2-(isocyanatoethyloxy) ethyl methacrylate, 2-acryloyloxyethyl isocyanate, 2-(isocyanatoethyloxy) ethyl acrylate, and 1,1-bis(acryloyloxymethyl)ethyl isocyanate.

3. The composition of claim 1 or 2, wherein a content of the compound (A) in the composition is 95.0% by mass or more.

4. A method of producing a composition, comprising steps of:

   producing a mixture comprising the compound (A) and the compound (B); and
   purifying the mixture by a distillation process at a reflux ratio of 2.0 to 4.0, a pressure of 1.0 to 10.0 kPa and a distillation temperature of 90 to 140°C,
   wherein the purifying step comprises:

      a vacuum breaking step of supplying a gas having a dew point of -30°C or less in a distillation apparatus after distillation by the distillation process and returning the pressure in the distillation apparatus to atmospheric pressure, wherein the gas comprises nitrogen gas oxygen gas at an oxygen concentration of 1 vol% or more and less than the critical oxygen concentration of compound (A); and
      a filling step of storing the purified product in the distillation apparatus after distillation in a container and filling a gas phase portion in the container with air having a dew point of -30°C or lower.

5. The method of producing a composition according to claim 4,

   wherein the mixture comprises more than 0.2 parts by mass of the compound (B) with respect to 100 parts by mass of the compound (A); and
   the purified product comprises 0.00002 to 0.2 parts by mass of the compound (B) with respect to 100 parts by mass of the compound (A).

6. A method of producing an unsaturated compound, comprising the steps of:

   mixing the composition according to any one of claims 1 to 3 with a compound having active hydrogen; and
   reacting the compound (A) contained in the composition with the compound having active hydrogen to obtain a reaction product.

7. The method of producing an unsaturated compound according to claim 6,
   wherein the compound having the active hydrogen is an alcohol, a thiol, an amine or a carboxylic acid.

8. The method of producing the unsaturated compound according to claim 6 or claim 7,
   wherein the reaction product is an unsaturated urethane compound, an unsaturated thiourethane compound, an unsaturated urea compound, or an unsaturated amide compound.

9. The method of producing an unsaturated compound according to claim 6 or claim 7,
   wherein the reaction product is any one selected from the group consisting of 2-butanone oxime-O-(carbamoylethyl-2-methacrylate), 2-butanone oxime-O-(carbamoylethyl-2-acrylate), 2-[(3,5-dimethylpyrazolyl)carbonylamino]ethyl methacrylate, and 2-[(3,5-dimethylpyrazolyl) carbonylamino]ethyl acrylate.


**Patentansprüche**

1. Zusammensetzung, umfassend:

   eine Verbindung (A) der allgemeinen Formel (1) und
   eine Verbindung (B),
   wobei die Verbindung (B) ein Oligomer ist, in dem zwei oder mehr Moleküle der Verbindungen (A) durch eine ethylenisch ungesättigte Gruppe jeder Verbindung (A) aneinander gebunden sind; und
   die Zusammensetzung 0,00002 bis 0,2 Massenteile der Verbindung (B) in Bezug auf 100 Massenteile der

Verbindung (A) enthält,

$$(R_1\text{-}COO)_n\text{-}R_2\text{-}(NCO)_m \qquad (1)$$

wobei in der allgemeinen Formel (1)
$R_1$ eine ethylenisch ungesättigte Gruppe mit 2 bis 7 Kohlenstoffatomen ist;
$R_2$ eine (m + n)-bindige Kohlenwasserstoffgruppe mit 1 bis 7 Kohlenstoffatomen ist und gegebenenfalls eine Ethergruppe aufweist; und
n und m jeweils eine ganze Zahl von 1 oder 2 sind.

2. Zusammensetzung nach Anspruch 1, wobei die Verbindung (A) mindestens eine Verbindung ist, die aus der Gruppe ausgewählt ist, die aus 2-Methacryloyloxyethylisocyanat, 2-(Isocyanatoethyloxy)ethylmethacrylat, 2-Acryloyloxyethylisocyanat, 2-(Isocyanatoethyloxy)ethylacrylat und 1,1-Bis(acryloyloxymethyl)ethylisocyanat besteht.

3. Zusammensetzung nach Anspruch 1 oder 2, wobei der Gehalt der Verbindung (A) in der Zusammensetzung 95,0 Massen-% oder mehr beträgt.

4. Verfahren zur Herstellung einer Zusammensetzung, mit den Schritten:

   Herstellen einer Mischung, die die Verbindung (A) und die Verbindung (B) enthält; und
   Reinigen der Mischung durch ein Destillationsverfahren bei einem Rückflussverhältnis von 2,0 bis 4,0, einem Druck von 1,0 bis 10,0 kPa und einer Destillationstemperatur von 90 bis 140 °C,
   wobei der Reinigungsschritt umfasst:

   einen Vakuumunterbrechungschritt, bei dem ein Gas mit einem Taupunkt von -30 °C oder weniger nach der Destillation durch das Destillationsverfahren in eine Destillationsapparatur eingeführt wird und der Druck in der Destillationsapparatur auf Atmosphärendruck zurückgeführt wird, wobei das Gas Stickstoffgas und Sauerstoffgas mit einer Sauerstoffkonzentration von 1 Vol.-% oder mehr und weniger als die kritische Sauerstoffkonzentration der Verbindung (A) umfasst; und
   einen Füllschritt, bei dem das gereinigte Produkt in der Destillationsapparatur nach der Destillation in einem Behälter gelagert und ein Gasphasenteil in dem Behälter mit Luft mit einem Taupunkt von -30°C oder weniger gefüllt wird.

5. Verfahren zur Herstellung einer Zusammensetzung nach Anspruch 4,

   wobei die Mischung mehr als 0,2 Masseteile der Verbindung (B) in Bezug auf 100 Massenteile der Verbindung (A) umfasst; und
   das gereinigte Produkt 0,00002 bis 0,2 Massenteile der Verbindung (B) in Bezug auf 100 Massenteile der Verbindung (A) enthält.

6. Verfahren zur Herstellung einer ungesättigten Verbindung, mit den Schritten:

   Mischen der Zusammensetzung nach einem der Ansprüche 1 bis 3 mit einer Verbindung mit aktivem Wasserstoff; und
   Umsetzen der in der Zusammensetzung enthaltenen Verbindung (A) mit der Verbindung mit aktivem Wasserstoff, um ein Reaktionsprodukt zu erhalten.

7. Verfahren zur Herstellung einer ungesättigten Verbindung nach Anspruch 6,
   wobei die Verbindung mit dem aktiven Wasserstoff ein Alkohol, ein Thiol, ein Amin oder eine Carbonsäure ist.

8. Verfahren zur Herstellung einer ungesättigten Verbindung nach Anspruch 6 oder 7,
   wobei das Reaktionsprodukt eine ungesättigte Urethanverbindung, eine ungesättigte Thiourethanverbindung, eine ungesättigte Harnstoffverbindung oder eine ungesättigte Amidverbindung ist.

9. Verfahren zur Herstellung einer ungesättigten Verbindung nach Anspruch 6 oder Anspruch 7,
   wobei das Reaktionsprodukt eines ist, das aus der aus 2-Butanonoxim-O-(carbamoylethyl-2-methacrylat), 2-Butanonoxim-O-(carbamoylethyl-2-acrylat), 2-[(3,5-Dimethylpyrazolyl)carbonylamino]ethylmethacrylat und 2-[(3,5-Dimethylpyrazolyl)carbonylamino]ethylacrylat bestehenden Gruppe ausgewählt ist.

**Revendications**

1. Composition comprenant :

   un composé (A) représenté par la formule générale (1) et
   un composé (B)
   dans laquelle le composé (B) est un oligomère dans lequel deux molécules ou plus des composés (A) sont liées les unes aux autres par un groupe à insaturation éthylénique de chaque composé (A) ; et
   la composition comprend 0,00002 à 0,2 partie en masse du composé (B) par rapport à 100 parties en masse du composé (A),

   $$(R_1\text{-COO})_n\text{-}R_2\text{-(NCO)}_m \qquad (1)$$

   dans laquelle dans la formule générale (1),
   $R_1$ est un groupe à insaturation éthylénique comportant 2 à 7 atomes de carbone ;
   $R_2$ est un groupe hydrocarboné à valence (m + n) comportant 1 à 7 atomes de carbone et comprend optionnellement un groupe éther ; et
   n et m représentent chacun un nombre entier de 1 ou 2.

2. Composition selon la revendication 1, dans laquelle le composé (A) est au moins un composé choisi dans le groupe constitué de l'isocyanate de 2-méthacryloyloxyéthyle, du méthacrylate de 2-(isocyanatoéthyloxy) éthyle, de l'isocyanate de 2-acryloyloxyéthyle, de l'acrylate de 2-(isocyanatoéthyloxy) éthyle et de l'isocyanate de 1,1-bis(acryloyloxyméthyl)éthyle.

3. Composition selon la revendication 1 ou 2, dans laquelle une teneur du composé (A) dans la composition est de 95,0 % en masse ou plus.

4. Procédé de production d'une composition, comprenant les étapes de :

   production d'un mélange comprenant le composé (A) et le composé (B) ; et
   purification du mélange par un procédé de distillation à un rapport de reflux de 2,0 à 4,0, une pression de 1,0 à 10,0 kPa et une température de distillation de 90 à 140 °C,
   dans lequel l'étape de purification comprend :

      une étape de cassage du vide consistant à fournir un gaz ayant un point de rosée de -30 °C ou moins dans un appareil de distillation après distillation par le procédé de distillation et à ramener la pression dans l'appareil de distillation à la pression atmosphérique, dans lequel le gaz comprend de l'azote gazeux et de l'oxygène gazeux à une concentration d'oxygène de 1 % en vol. ou plus et inférieure à la concentration d'oxygène critique du composé (A) ; et
      une étape de chargement consistant à stocker le produit purifié dans l'appareil de distillation après distillation dans un récipient et à charger une portion en phase gazeuse dans le récipient avec de l'air ayant un point de rosée de -30 °C ou moins.

5. Procédé de production d'une composition selon la revendication 4,

   dans lequel le mélange comprend plus de 0,2 partie en masse du composé (B) par rapport à 100 parties en masse du composé (A) ; et
   le produit purifié comprend 0,00002 à 0,2 partie en masse du composé (B) par rapport à 100 parties en masse du composé (A).

6. Procédé de production d'un composé insaturé, comprenant les étapes de :

   mélange de la composition selon l'une quelconque des revendications 1 à 3 avec un composé renfermant de l'hydrogène actif ; et
   réaction du composé (A) contenu dans la composition avec le composé renfermant de l'hydrogène actif pour obtenir un produit réactionnel.

7. Procédé de production d'un composé insaturé selon la revendication 6,

dans lequel le composé renfermant l'hydrogène actif est un alcool, un thiol, une amine ou un acide carboxylique.

8. Procédé de production du composé insaturé selon la revendication 6 ou la revendication 7,
   dans lequel le produit réactionnel est un composé d'uréthane insaturé, un composé de thiouréthane insaturé, un composé d'urée insaturé ou un composé d'amide insaturé.

9. Procédé de production d'un composé insaturé selon la revendication 6 ou la revendication 7,
   dans lequel le produit réactionnel est l'un quelconque choisi dans le groupe constitué de l'oxime-O-(carbamoyléthyl-2-méthacrylate) de 2-butanone, de l'oxime-O-(carbamoyléthyl-2-acrylate) de 2-butanone, du méthacrylate de 2-[(3,5-diméthylpyrazolyl)carbonylamino]éthyle et de l'acrylate de 2-[(3,5-diméthylpyrazolyl)carbonylamino]éthyle.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2821544 A
- JP H06322051 A
- JP H06187822 A
- JP S60234582 A
- JP 60234583 A
- JP H06184256 A
- WO 2011074503 A
- JP 4273531 B
- JP 4823546 B
- JP 2007008828 A

**Non-patent literature cited in the description**

- Polymer degradation mechanism and stabilization technology. CMC Technical Library. CMC Publishing Co., Ltd, April 2005, 168